# EUROPÄISCHE PATENTANMELDUNG

(11) **EP 0 407 346 A2**
(43) Veröffentlichungstag der Anmeldung: **09.01.1991**
(21) Anmeldenummer: 90810488.8
(22) Anmeldetag: 28.06.1990
(51) Int. Cl.: C07D 213/643, C07D 213/75, A01N 43/40, A01N 47/28, C07D 213/73, C07D 213/74, C07D 213/70

(54) **Aminopyridine**

(30) Priorität: 07.07.1989 CH 2533/89
(71) Anmelder: CIBA-GEIGY AG, CH-4002 Basel (CH)
(72) Erfinder: Pascual, Alfons, Dr., CH-4053 Basel (CH)

(57) **Zusammenfassung**

Neue 3-Amino-2,4-dialkylpyridin-Derivate 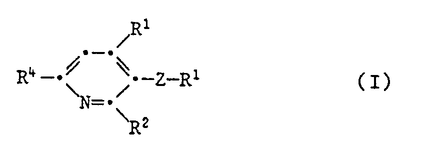 worin
R¹ und R² unabhängig voneinander Ci-C₆-Alkyl,
R³ C₁-C₁₂-Alkyl, C₃-C₆-Cycloalkyl, durch Cs-C₆-Cycloalkyl substituiertes C₁-C₄-Alkyl oder durch C₁-C₄-Alkyl substituiertes Cs-C₆-Cycloalkyl,
R⁴ Wasserstoff, Halogen, Cᵢ-C₆-Alkyl, Phenoxy oder einfach oder zweifach durch Halogen, C₁-C₄-Alkyl, C₁-C₄-Alkoxy, C₁-C₄-Alkylthio, C₁-C₄-Alkylamino, Di-C₁-C₄-alkylamino, C₁-C₄-Alkylcarbonylamino, C₁-C₄-Alkylcarbonyl, Benzoyl, Nitro, Cyan, C₁-C₄-Alkoxycarbonyl, C₁-C₄-Halogenalkyl oder C₁-C₄-Halogenalkoxy substituiertes Phenoxy und
Z ein Brückenglied -NH-CS-NH-, -N=C(SR⁵)-NH-oder -N=C=N- bedeuten, wobei R⁵ für C₁-C₆-Alkyl oder C₃-Cs-Alkenyl steht, können als Schädlingsbekämpfungsmittel eingesetzt werden. Vorzugsweise können Insekten und Arachniden bekämpft werden.

## Beschreibung

Die vorliegende Erfindung betrifft neue Derivate von 3-Amino-2,4-dialkylpyridinen, Verfahren und Zwischenprodukte zu ihrer Herstellung, Schädlingsbekämpfungsmittel, welche diese Verbindungen enthalten, sowie ihre Verwendung bei der Kontrolle von Schädlingen.

Die erfindungsgemässen 3-Amino-2,4-dialkylpyridine entsprechen der Formel I 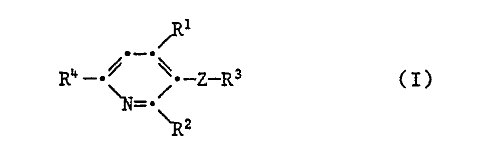 worin
R' und R² unabhängig voneinander C₁-C₆-Alkyl,
R³ C₁-C₁₂-Alkyl, C₃-C₆-Cycloalkyl, durch C₃-C₆-Cycloalkyl substituiertes C₁-C₄-Alkyl oder durch C₁-C₄-Alkyl substituiertes C₃-C₆-Cycloalkyl,
R⁴ Wasserstoff, Halogen, C₁-C₆-Alkyl, Phenoxy oder einfach oder zweifach durch Halogen, C₁-C₄-Alkyl, C₁-C₄-Alkoxy, C₁-C₄-Alkylthio, Cₗ-C₄-Alkylamino, Di-C₁-C₄-alkylamino, C₁-C₄-Alkylcarbonylamino, C₁-C₄-Alkylcarbonyl, Benzoyl, Nitro, Cyan, C₁-C₄-Alkoxycarbonyl, C₁-C₄-Halogenalkyl oder C₁-C₄-Halogenalkoxy substituiertes Phenoxy und
Z ein Brückenglied -NH-CS-NH-, -N = C(SR⁵)-NH-oder -N = C = N- bedeuten, wobei R⁵ für C₁-C₆-Alkyl oder C₃-C₅-Alkenyl steht.

Aus der Literatur ist die Klasse der Pyridylguanidine als pharmazeutisch wirksame Blutdrucksenker aus der DE-OS 2 557 438 bekannt. Pyridylcarbodiimide werden äls Zwischenprodukte zur Herstellung dieser Wirkstoffe beschrieben.

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor und Chlor bevorzugt sind. Halogen ist dabei als selbständiger Substituent oder als Teil eines Substituenten zu verstehen wie im Halogenalkyl oder Halogenalkoxy.

Die als Substituenten in Betracht kommenden Alkyl-, Alkylthio- und Alkoxyreste können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl und ihre Isomeren genannt. Als geeignete Alkoxyreste seien unter anderem genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio.

Sind die als Substituenten in Betracht kommenden Alkyl-, Alkoxyl-, oder Phenylgruppen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das ein- bis dreifach durch Fluor, Chlor und/oder Brom substituierte Methyl wie beispielsweise CHF₂ oder CF₃; das einbis fünffach durch Fluor, Chlor und/oder Brom substituierte Aethyl wie zum Beispiel CH₂CF₃, CF₂CFs, CF₂CCl₃, CF₂CHCI₂, CF₂CHF₂, CF₂CFCI₂, CF₂CHBr₂, CF₂CHCIF, CF₂CHBrF oder CCIFCHCIF; das einbis siebenfach durch Fluor, Chlor und/oder. Brom substituierte Propyl oder Isopropyl wie z.B. CH₂CHBrCH₂Br, CF₂CHFCF₃, CH₂CF₂CF₃ oder CH(CF₃)₂; das ein- bis neunfach durch Fluor, Chlor und/oder Brom substituierte Butyl oder eines seiner Isomeren wie zum Beispiel CF(CF₃)CHFCF₃ oder CH₂-(CF₂)₂CFs.

Bei den als Substituenten in Betracht kommenden Cycloalkylresten handelt es sich beispielsweise um Cyclopropyl, Cyclobutyl, Cyclopentyl oder Cyclohexyl.

Beispiele für Alkoxycarbonyl- und Alkylcarbonylreste sind Methoxycarbonyl, Aethoxycarbonyl, Propoxy.carbonyl, Isopropoxycarbonyl oder Butoxycarbonyl. Alkylcarbonyl steht beispielsweise für Acetyl, Propionyl, Butyryl oder Valeryl, sowie Isomere davon.

Unter den Verbindungen der Formel 1 sind solche Untergruppen herauszuheben, in denen entweder
a) R' und R² die gleiche Bedeutung haben, oder
b) R³ für verzweigtes C₃-C₅-Alkyl steht, oder
c) R⁴ für durch Halogen, C₁-C₄-Alkyl oder C₁-C₄-Alkoxy substituietes Phenoxy steht, oder
d) R⁵ für C₁-C₄-Alkyl steht.

Unter den Verbindungen der Untergruppe a) sind solche bevorzugt, worin R¹ und R² für Isopropyl stehen; unter denen der Untergruppe b) solche, worin R³ Isopropyl, tert.Butyl oder 1,1-Dimethylpropyl bedeutet; und unter denen der Untergruppe c) solche, worin R⁴ für Chlorphenoxy, Fluorphenoxy, Methoxyphenoxy, tert.Butylphenoxy oder Dichlorphenoxy steht.

Eine besonders bevorzugte Untergruppe von Verbindungen der Formel I ist dadurch gekennzeichnet, dass R' und R² die gleiche Bedeutung haben, R³ für verzweigtes C₃-C₅-Alkyl und R⁴ für durch Halogen, C₁-C₄-Alkyl, Di-C₁-C₄-alkylamino oder C₁-C₄-Alkoxy substituiertes Phenoxy stehen.

Insbesondere ist wegen der vorteilhaften Wirksamkeit gegen Schädlinge die Gruppe von Verbindungen der Formel I herauszustreichen, worin R¹ und R² für Isopropyl, R³ für Isopropyl, tert.Butyl oder 1,1-Dimethylpropyl und R⁴ für Chlorphenoxy, Fluorphenoxy, Dichlorphenoxy, Methoxyphenoxy oder tert.Butylphenoxy stehen.

Als bevorzugte Einzelverbindungen der Formel I sind zu nennen:
1-tert.Butyl-3-[2,4-diisopropyl-6-(4-chlorphenoxy)-pyrid-3-yl]-thioharnstoff,
1-tert.Butyl-3-[2,4-diisopropyl-6-(4-chlorphenoxy)-pyrid-3-yl]-S-methyl-isothioharnstoff,
1-tert.Butyl-3-[2,4-diisopropyl-6-(4-chlorphenoxy)-pyrid-3-yl]-carbodiimid,
1-tert.Butyl-3-[2,4-diisopropyl-6-(4-methoxyphenoxy)-pyrid-3-yl]-thioharnstoff,
1-tert.Butyl-3-[2,4-diisopropyl-6-(4-methoxyphenoxy)-pyrid-3-yl]-S-methyl-isothioharnstoff und
1-tert.Butyl-3-[2,4-diisopropyl-6-(4-methoxyphenoxy)-pyrid-3-yl]-carbodiimid.

Die Verbindungen der Formel I, in denen Z-R³ für -N=C(SR^{s})-NH-_{R}³ steht, können in den beiden tautomeren Formen 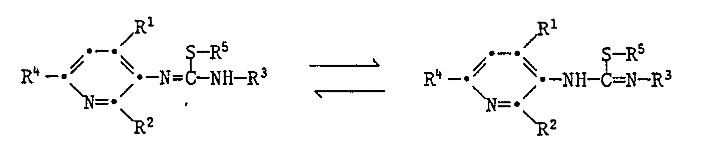 vorliegen. Die Erfindung umfasst sowohl die einzelnen Tautomeren als auch Tautomerengemische.

Die erfindungsgemässen Verbindungen der Formel I können in Analogie zu bekannten Verfahren hergestellt werden. Beispielsweise erhält man die Verbindung der Formel I, indem man
a) ein Isothiocyanat der Formel II 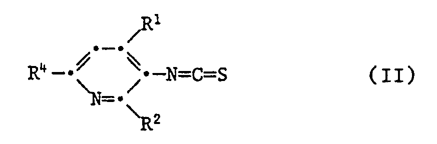 worin R', R² und R⁴ die unter Formel I gegebenen Bedeutungen haben, mit einem primären Amin der Formel III H₂N-R³ (III) worin R³ die unter Formel I gegebene Bedeutung hat zum Thioharnstoff der Formel la 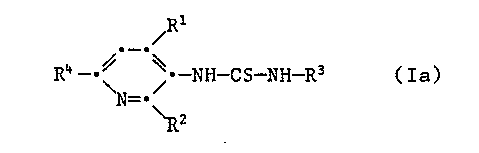 umsetzt und gewünschtenfalls entweder
b) den erhaltenen Thioharnstoff der Formel la mit einem Alkylierungsmittel der Formel IV ^{Y-R5} (IV) worin R⁵ die unter Formel I gegebene Bedeutung hat und Y für eine Abgangsgruppe steht, in den Isothioharnstoff der Formel Ib 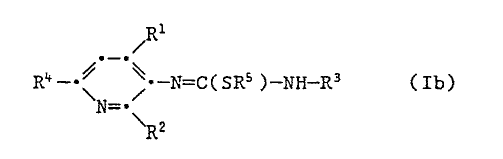 umsetzt oder
c) den erhaltenen Thioharnstoff der Formel la durch Abspalten von Schwefelwasserstoff in das Carbodiimid der Formel Ic 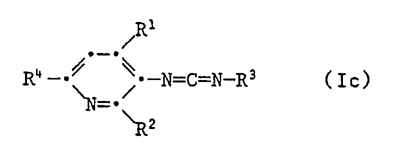 überführt.

Das Verfahren a) wird üblicherweise unter normalem Druck, und in Gegenwart eines organischen Lösungs- oder Verdünnungsmittels durchgeführt. Die Temperatur beträgt dabei zwischen 0°C und +150°C, vorzugsweise +10°C bis +70°C. Als Lösungs- oder Verdünnungsmittel eignen sich beispielsweise Aether und ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylen chlorid, Tetrachlorkohlenstoff oder Chlorbenzol; Nitrile, wie Acetonitril oder Propionitril; Ketone, wie Aceton, Methyläthylketon, Methylisopropylketon, Methylisobutylketon oder Cyclohexanon.

Das Verfahren b) wird zweckmässigerweise in einem inerten organischen Lösungsmittel und unter leicht erhöhtem oder normalem Druck durchgeführt. Die Temperatur beträgt dabei zwischen +10°C und +250°C, vorzugsweise Siedetemperatur des verwendeten Lösungsmittels oder +50°C bis +₁₅0°C. Geeignete Lösungs- oder Verdünnungsmittel sind zum Beispiel Aether oder ätherartige Verbindungen, wie Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon, Alkohole oder Dimethylformamid.

Das Verfahren c) wird zweckmässigerweise in einem aprotischen organischen Lösungs- oder Verdünnungsmittel unter normalem Druck durchgeführt. Die Temperatur beträgt dabei zwischen 0°C und +₁₅0°C, vorzugsweise +10°C bis +50°C. Geeignete Lösungs- oder Verdünnungsmittel sind zum Beispiel Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon oder Cyclohexanon. Die Abspaltung von Schwefelwasserstoff erfolgt nach in der Literatur beschriebenen Arbeitsweisen (Chemistry Letters 1977, p. 575-76; Tetrahedron Letters 1985, p. 1661-64; Ber. Dtsch. Chem. Ges. 6 , 1873, p. 1398; Bull. Soc. Chim. 1956, p. 1360). Als Abspaltungs-Reagenzien werden dabei unter anderem HgO, bestimmte Pyridinium Salze, Chloressigsäureester, Cyanursäurechlorid, p-Toluolsulfonsäurechlorid oder bestimmte Phosphorsäureester-Derivate verwendet.

Die Isothiocyanate der Formel II können nach im Prinzip bekannten Methoden hergestellt werden, indem man ein 3-Aminopyridin der Formel V 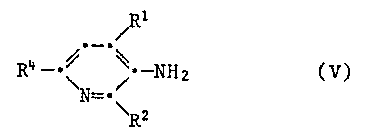 worin R', R² und R⁴ die für Formel I angegebene Bedeutung haben, entweder
a) mit Schwefelkohlenstoff in Gegenwart eines tertiären Amins in Pyridin umsetzt und das Reaktionsprodukt mit N,N'-Dicyclohexylcarbodiimid in Pyridin behandelt, wie in dem in Chem. Ber. 101 , 1746 (1968) beschriebenen Verfahren, oder
b) mit Schwefelkohlenstoff in Gegenwart eines tertiären Amins umsetzt, das Reaktionsprodukt mit einem Methylierungsmittel wie Methyljodid oder Dimethylsulfat methyliert und bei einer Temperatur zwischen +80°C und +250°C, insbesondere zwischen +120°C und +160°C, wie beispielsweise bei +140°C thermolysiert, wie in dem in J. Chem. Soc. 1956 , 1644 beschriebenen Verfahren.

Als tertiäre Amine kommen für die grosstechnische Synthese vorzugsweise solche Amine zum Einsatz, die gleichzeitig als Lösungsmittel dienen können, wie Triäthylamin und Pyridin. In Labormassstab eignen sich aber auch tertiäre Amine wie 1,5-Diazabicyclo[4.3.0]non-5-en oder 1.8-Diazabicyclo[5.4.0]undec-7-en.

Die 3-Aminopyridine der Formel V können ebenfalls nach an sich bekannten Verfahren erhalten werden. Auf besonders einfache Weise werden die 3-Aminopyridine der Formel V erhalten, indem man ein 3-Nitropyridin der Formel VI 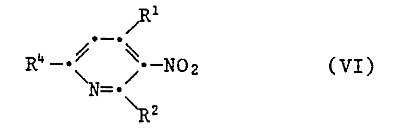 worin R', R² und R⁴ die unter Formel I gegebenen Bedeutungen haben, katalytisch mit Wasserstoff reduziert.

Die katalytische Reduktion wird in üblicher Weise durch Reaktion mit gasförmigem Wasserstoff in Gegenwart eines geeigneten Katalysators mit Vorteil in einem inerten Lösungsmittel, durchgeführt. Die Reduktionsreaktion kann sowohl bei Normaldruck als auch unter erhöhtem Druck bis zu 20 bar ausgeführt werden. Die Reaktionstemperaturen liegen zwischen +10°C und +40°C.

Geeignete Katalysatoren sind Platin-, Palladium- oder Nickelkatalysatoren, wie Platin, Platinmoor, Platin/BaS0₄, Palladium, Palladium/Kohle oder Raney-Nickel. Als Lösungsmittel können Alkohole, wie Methanol, Aethanol oder Isopropanol; Carbonsäureester, wie Aethylacetat; oder Aether wie Diäthyläther, Tetrahydrofuran oder Dioxan verwendet werden. Für den Fall, dass R⁴ für Halogen steht, kann bei der Reduktion durch geeignete Wahl der Reaktionsbedingungen entweder das 3-Aminopyridin unter Erhalt des 6-Halogenatoms erzeugt werden, oder es kann im gleichen Reaktionsschritt das Halogenatom entfernt werden, sodass man diejenigen 3-Aminopyridine der Formel V erhält, worin R⁴ Wasserstoff bedeutet.

Die 3-Nitropyridine der Formel VI, worin R⁴ für Chlor oder Brom steht, werden durch Umsetzen von 6-Hydroxy-3-nitropyridinen der Formel VII 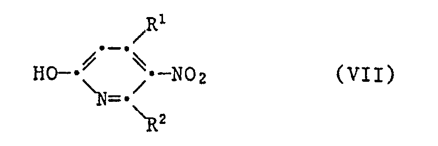 worin R' und R² die unter Formel I gegebenen Bedeutungen haben, mit Chlorierungs- oder Bromierungsmittel wie Phosphorpentachlorid oder Phosphorpentabromid, erhalten. Aus den so hergestellten 6-Chlor-oder 6-Brom-3-nitropyridinen der Unterformel Vla 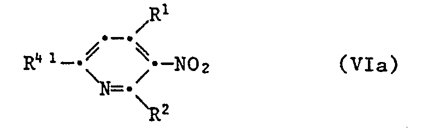 worin R' und R² die unter Formel I gegebenen Bedeutungen haben und R⁴¹ für Chlor oder Brom steht, werden die 6-Phenoxy-3-nitropyridine der Unterformel Vlb 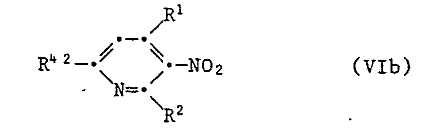 worin R¹ und R² die unter Formel I gegebenen Bedeutungen haben und R⁴² für Phenoxy oder einfach oder zweifach durch Halogen, C₁-C₄-Alkyl, C₁-C₄-Alkoxy, C₁-C₄-Alkylthio, Cₗ-C₄-Alkylamino, Di-C₁-C₄-alkylami- no, Cₗ-C₄-Alkylaminocarbonyl, C₁-C₄-Alkylcarbonyl, Benzoyl, Nitro, Cyan, C₁-C₄-Alkoxycarbonyl, C₁-C₄-Halogenalkyl oder C₁-C₄-Halogenalkoxy substituiertes Phenoxy steht, erhalten, indem man in Gegenwart einer starken Base die Verbindung Via mit einem Phenol der Formel VIII _{R}4²⁻H (VIII) umsetzt.

Die Substitutionsreaktion (Via → Vlb) kann sowohl unter üblichen Phasentransferbedingungen in einem zweiphasigen Reaktionsmedium als auch in einer einphasigen Reaktion durchgeführt werden. Beim typischen Phasentransferverfahren werden die Reaktanden Via und VIII in einem aromatischen Lösungsmittel wie Benzol, Toluol oder Xylol gelöst, ein Phasentransferkatalysator wie beispielsweise 18-Crown-6, Tetrabtuylammoniumchlorid oder Triäthylbutylammoniumchlorid zugesetzt und als zweite Phase eine konzentrierte wässrige Alkalilauge, zum Beispiel 50%ige Natronlauge, zugefügt. Die Reaktionstemperaturen liegen zwischen +20°C und dem Siedepunkt des Gemisches. Beim einphasigen Reaktionsablauf werden VIa, VIII und Alkalibase in einem polaren Lösungsmittel wie Dimethylsulfoxid, Dimethylformamid, Methanol, Aethanol oder Isopropanol gelöst und vorzugsweise zum Siedepunkt erwärmt.

Die 6-Hydroxy-3-nitropyridine der Formel VII können analog zum folgenden Schema hergestellt werden.

### Schema 1:

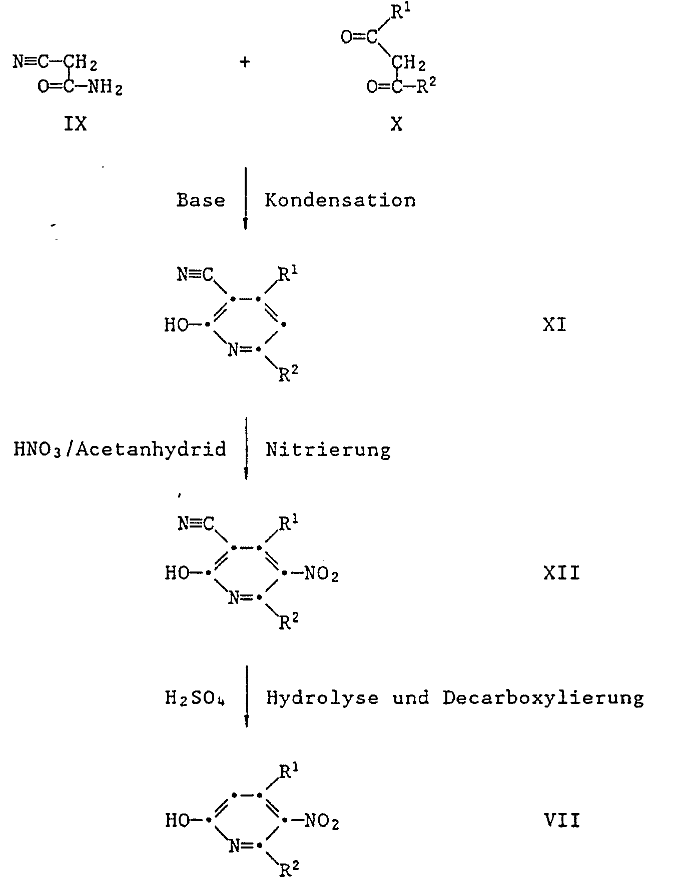

R' und R² haben die unter Formel I gegebenen Bedeutungen. Reaktionsbedingungen und Reagenzien werden wie in analogen literaturbekannten Verfahrensschritten gewählt.

Die Zwischenprodukte der Formel II sind neu. Sie werden speziell für die Synthese der Wirkstoffe der Formel I entwickelt. Sie bilden daher einen Gegenstand der vorliegenden Erfindung. Ebenfalls neu sind die Zwischenprodukte der Formeln V und VI mit Ausnahme der beiden Einzelverbindungen, worin R', R² und R⁴ jeweils Methyl bedeuten. Diese neuen Zwischenprodukte sind ebenfalls Gegenstand dieser Erfindung.

Die Zwischenprodukte der Formeln III, IV und VIII sind zum grossen Teil bekannt und im Handel erhältlich, oder sie lassen sich nach bekannten Methoden herstellen.

Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus-und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beipsielsweise bei einer Häutung, oder in einer verminderten Eiablage und/oder Schlupfrate zeigen. Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung der Orthoptera zum Beispiel
Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.;
aus der Ordnung der Psocoptera zum Beispiel
Liposcelis spp.;
aus der Ordnung der Anoplura zum Beispiel
Haematopinus spp., Linognathus spp. Pediculus spp., Pemphigus spp. und Phylloxera spp.;
aus der Ordnung der Mallophaga zum Beispiel
Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii; aus der Ordnung der Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.; aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.; aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen Cs bis C₁₂, wie Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation-und/oder anionaktive Tenside mit guten, Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren (C1α-C22), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium-oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"1985 International Mc Cutcheon's Emulsifiers & Detergents", Glen Rock NJ USA, 1985",
H. Stache, "Tensid-Taschenbuch", 2. Aufl., C. Hanser Verlag München, Wien 1981,
M. and J. Ash. "Encyclopedia of Surfactants", Vol. 1-111, Chemical Publishing Co., New York, 1980-1981.

Die pestiziden Zubereitungen enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen: (% = Gewichtsprozent)
Emulgierbare Konzentrate
Aktiver Wirkstoff: 1 bis 20 %, bevorzugt 5 bis 10 %
oberflächenaktives Mittel: 5 bis 30 %, vorzugsweise 10 bis 20 %
flüssiges Trägermittel: 50 bis 94 %, vorzugsweise 70 bis 85 %
Stäube:
Aktiver Wirkstoff: 0,1 bis 10 %, vorzugsweise 0,1 bis 1 %
festes Trägermittel: 99,9 bis 90 %, vorzugsweise 99,9 bis 99 %
Suspension-Konzentrate
Aktiver Wirkstoff: 5 bis 75 %, vorzugsweise 10 bis 50 %
Wasser: 94 bis 24 %, vorzugsweise 88 bis 30 %
oberflächenaktives Mittel: 1 bis 40 %, vorzugsweise 2 bis 30 %
Benetzbare Pulver
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermaterial: 5 bis 95 %, vorzugsweise 15 bis 90 %
Granulate
aktiver Wirkstoff: 0,5 bis 30 %, vorzugsweise 3 bis 15 %
festes Trägermittel: 99,5 bis 70 %, vorzugsweise 97 bis 85 %

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

### Herstellungsbeispiele

### Beispiel H1: 2,4-Diisopropyl-6-(4-chlorphenoxy)-3-nitropyridin

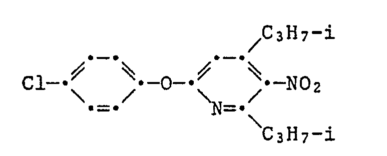

0,77 g 4-Chlorphenol und 1,00 g Kaliumcarbonat werden in 6 ml Dimethylsulfoxid suspendiert und für 1 Stunde lang auf +60°C erwärmt; anschliessend lässt man eine Lösung von 1,46 g 6-Chlor-2,4-diisopropyl-3-nitropyridin in 4 ml Dimethylsulfoxid zutropfen. Nach beendeter Zugabe wird der Ansatz unter kräftigem Rühren für 1 Stunde auf + 120 C erwärmt. Nach dem Abkühlen wird das Reaktionsgemisch auf 100 ml Wasser und 50 ml Toluol gegossen und anschliessend mit Kochsalz gesättigt. Die entstandene Suspension wird über Diatomeenerde abfiltriert und das Filtrat mit Toluol extrahiert. Die organische Phase wird über Magnesiumsulfat getrocknet und das Lösungsmittel abgedampft. Der Rückstand wird mittels Säulenchromatographie an Kieselgel (Laufmittel: Aethylacetat/Hexan 1:30) gereinigt. Man erhält 2, 4-Diisopropyl-6-(4- chlorphenoxy)-3-nitropyridin als hellgelbes Kristallpulver, Smp. 102-104 C.

### Beispiel H2: 2,4-Diisopropyl-6-(4-chlorphenoxy)-3-aminopyridin

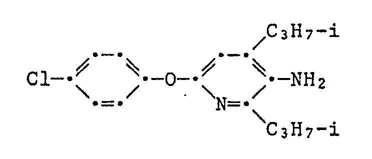

11,9 g 2,4-Diisopropyl-6-(4-chlorphenoxy)-3-nitropyridin werden in 120 ml Tetrahydrofuran gelöst und nach Zugabe von 12 g Raney-Hickel (in Aethanol suspendiert) bei +30°C bis +35°C bei Hormaldruck hydriert. Das Reaktionsgemisch wird über Diatomeenerde abfiltriert und das Filtrat eingeengt. Der Rückstand wird mittels Säulenchromatographie an Kieselgel (Laufmittel: Aethylacetat/Hexan 1:3) gereinigt. Man erhält 2,4-Diisopropyl-6-(4-chlorphenoxy)-3-aminopyridin als hellgelbes Kristallpulver, Smp. 86-88 C.

### Beispiel H3: 2,4-Diisopropyl-3-aminopyridin

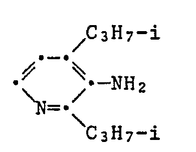

5,0 g 6-Chlor-2,4-diisopropyl-3-nitropyridin werden in 100 ml Tetrahydrofuran gelöst und nach Zugabe von 2,75 g Triäthylamin und 7,5 g (5%-igem Palladium/Kohle-Katalysator bei +20°C bis +25°C bei Normaldruck hydriert. Das Reaktionsgemisch wird über Diatomeenerde abfiltriert und das Filtrat eingeengt. Der Rückstand wird säulenchromatographisch an Kieselgel gereinigt (Laufmittel: Aethylacetat/Hexan 1:3). Man erhält 2,4-Diisopropyl-3-aminopyridin als wachsartigen Festkörper, Smp. 35-39°C.

### Beispiel H4: 6-Chlor-2,4-diisopropyl-3-aminopyridin

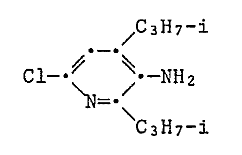

30,0 g 6-Chlor-2,4-diisopropyl-3-nitropyridin werden in 450 ml Tetrahydrofuran gelöst und nach Zugabe von 30,0 g Raney-Hickel (in Aethanol suspendiert) bei + 30 C bis + 35 °C bei 20 bar Wasserstoffdruck hydriert. Das Reaktionsgemisch wird über Diatomeenerde abfiltriert und das Filtrat eingeengt. Der Rückstand wird säulenchromatographisch an Kieselgel gereinigt (Laufmittel: Aethylacetat/Hexan 1:3). Man erhält 6-Chlor-2,4-diisopropyl-3-aminopyridin als helles Kristallpulver, Smp. 53-56°C.

### Beispiel H5: 2,4-Diisopropyl-6-(4-chlorphenoxy)-pyrid-3-ylisothiocyanat

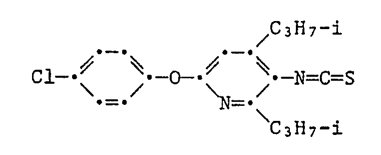

Eine Lösung von 5,0 g Triäthylamin in 10 ml Pyridin wird auf -5°C bis -10°C abgekühlt und tropfenweise 20 ml Schwefelkohlenstoff versetzt. Bei -10°C lässt man eine Lösung von 15,0 g 2,4-Diisopropyl-6-(4-chlorphenoxy)-3-aminopyridin in 27 ml Pyridin langsam zutropfen. Für 1 Stunde wird bei -10°C weiter gerührt. Anschliessend wird eine Lösung von 10,0 g N,N'-Dicyclohexylcarbodiimid in 10 ml Pyridin zugegeben. Nach weiteren 3 Stunden bei -10°C wird die Mischung für 18 Stunden bei Raumtemperatur gerührt. Das Reaktionsgut wird eingedampft und der Rückstand mit Hexan versetzt. Die Hexan-Lösung wird abfiltriert und eingedampft. Man erhält 2, 4-Diisopropyl-6-(4-chlorphenoxy)-pyrid-3-ylisothiocyanat in Form eines gelben Oeles, das ohne weitere Reinigung für die nächste Reaktion verwendet wird, IR(CCI₄): 2090,1490, cm-'.

### Beispiel H6: 1-tert.Butyl-3-[2,4-diisopropyl-6-(4-chlorphenoxy)-pyrid-3-yl]-thioharnstoff

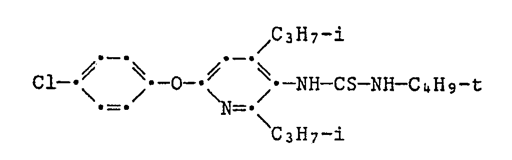

8,15 g 2,4-Diisopropyl-6-(4-chlorphenoxy)-pyrid-3-ylisothiocyanat werden mit 30 ml Toluol verdünnt und tropfenweise mit 2,0 g tert.Butylamin versetzt. Anschliessend wird bei ca. +60°C für 2 Stunden weiter gerührt. Das Reaktionsgemisch wird eingeengt und der Rückstand mit Hexan versetzt. Der entstandene Festkörper wird abfiltriert und mit Hexan nachgewaschen. Man erhält 1-tert.Butyl-3-[2,4-diisopropyl-6-(4- chlorphenoxy)-pyrid-3-yl]-thioharnstoff in Form farbloser Kristalle, Smp. 165-167" C.

### Beispiel H7: 1-tert.Butyl-3-[2,4-diisopropyl-6-(4-chlorphenoxy)-pyrid-3-yl]-S-methyl-isothioharnstoff

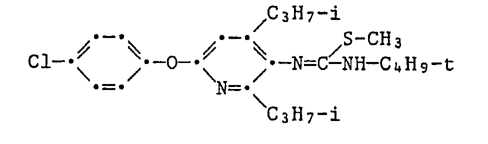

2,50 g 1-tert.Butyl-3-[2,4-diisopropyl-6-(4-chlorphenoxy)-pyrid-3-yl]-thioharnstoff in 30 ml Aethanol werden bei Raumtemperatur mit 2,0 g Methyljodid versetzt und für 5 Stunden auf + 75 C erwärmt. Anschliessend wird die Mischung eingedampft, der Rückstand in Methylenchlorid aufgenommen und zweimal mit verdünnter Natriumbicarbonatlösung gewaschen. Die organische Phase wird über Magnesiumsulfat getrocknet und das Lösungsmittel abgedampft. Das Rohprodukt wird mittels Säulenchromatographie an Kieselgel (Laufmittel: Aethylacetat/Hexan 1:3) gereinigt. Man erhält 1-tert.Butyl-3-[2,4-diisopropyl-6-(4-chlorphenoxy)-pyrid-3-yl]-S-methyl-isothioharnstoff in Form eines farblosen Kristallpulvers, Smp. 95-100°C.

### Beispiel H8: 1-tert.Butyl-3-[2,4-diisopropyl-6-(4-chlorphenoxy)-pyrid-3-yl]-carbodiimid

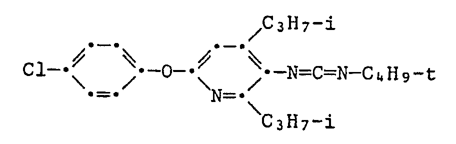

3,0 g 1-tert.Butyl-3-[2,4-diisopropyl-6-(4-chlorphenoxy)-pyrid-3-yl]-thioharnstoff und 2,1 g 2-Chlor-1-methylpyridiniumjodid werden in 20 ml Acetonitril vorgelegt und bei Raumtemperatur mit einer Lösung von 1,6 g Triäthylamin in 7 ml Acetonitril versetzt. Anschliessend wird die Reaktion für 1 Stunde bei ca. +65°C verrührt. Nach dem Abdampfen des Lösungsmittels wird der Rückstand in Hexan/Wasser aufgenommen. Die organische Phase wird über Magnesiumsulfat getrocknet und das Lösungsmittel am Vakuum entfernt. Das Rohprodukt wird mittels Säulenchromatographie an Kieselgel (Laufmittel: AethylacetatlHexan 1:5) gereinigt. Man erhält 1-tert.Butyl-3-[2,4-diisopropyl-6-(4-chlorphenoxy)-pyrid-3-yl]-carbodiimid als farbloses Oel, welches nach einigen Stunden erstarrt. Smp 46-55°C.

In analoger Weise erhält man die in den folgenden Tabellen 1 bis 6 aufgelisteten Zwischenprodukte und erfindungsgemässen Wirkstoffe.

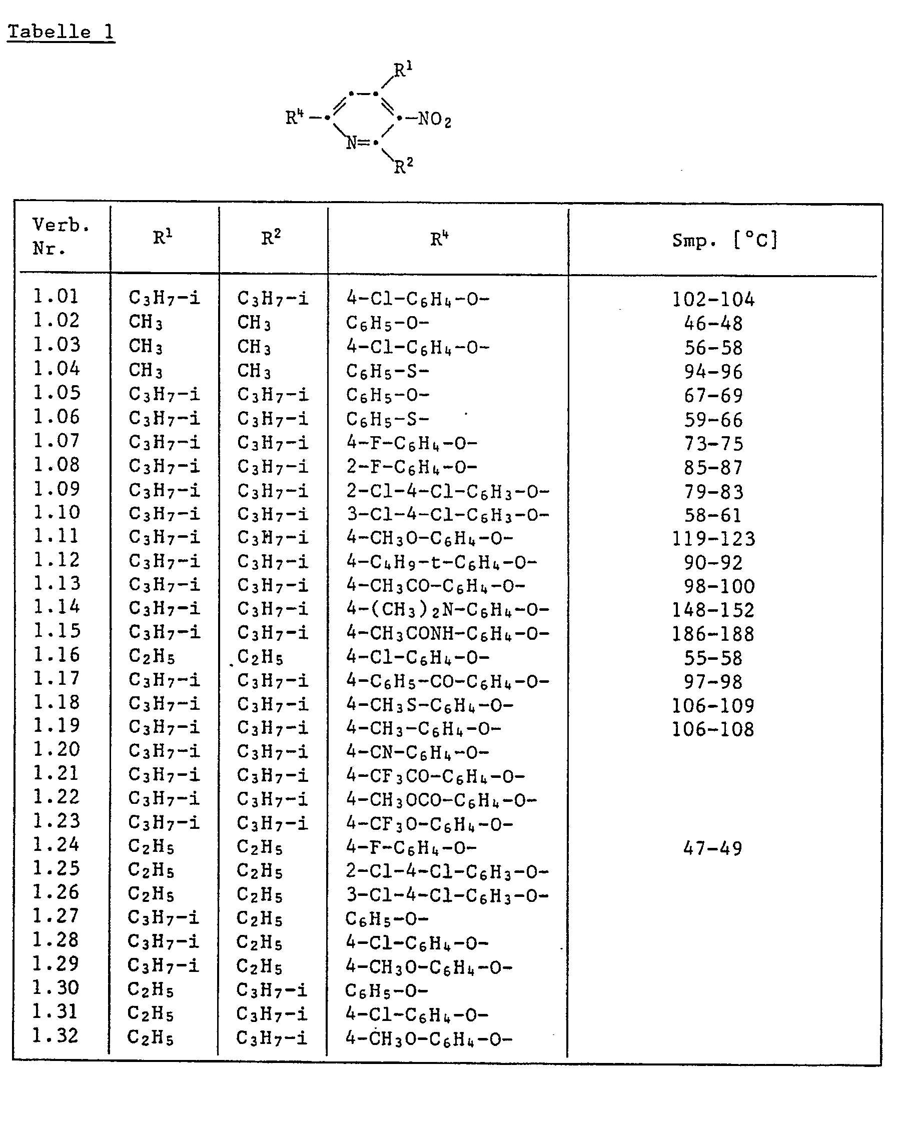

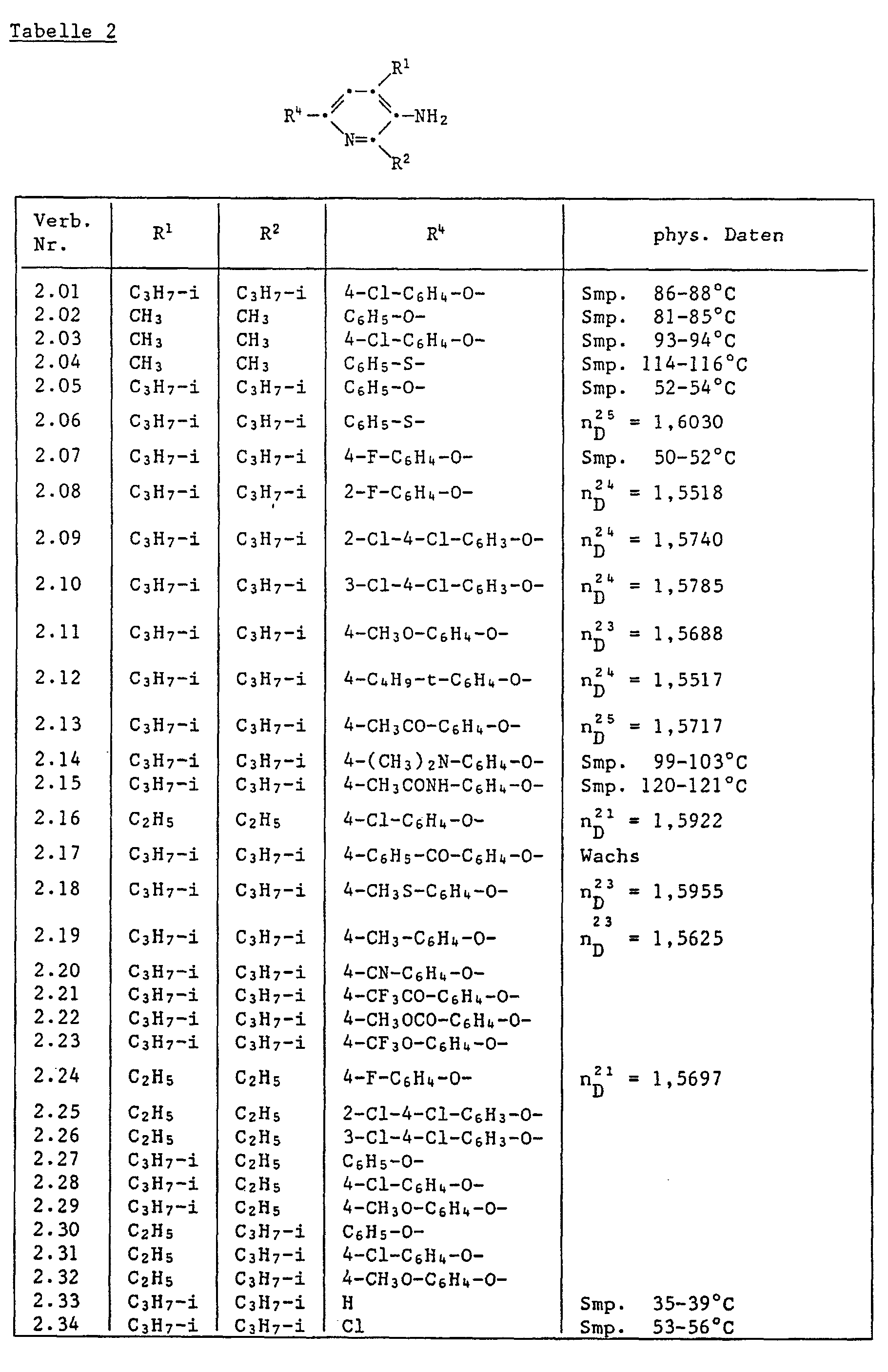

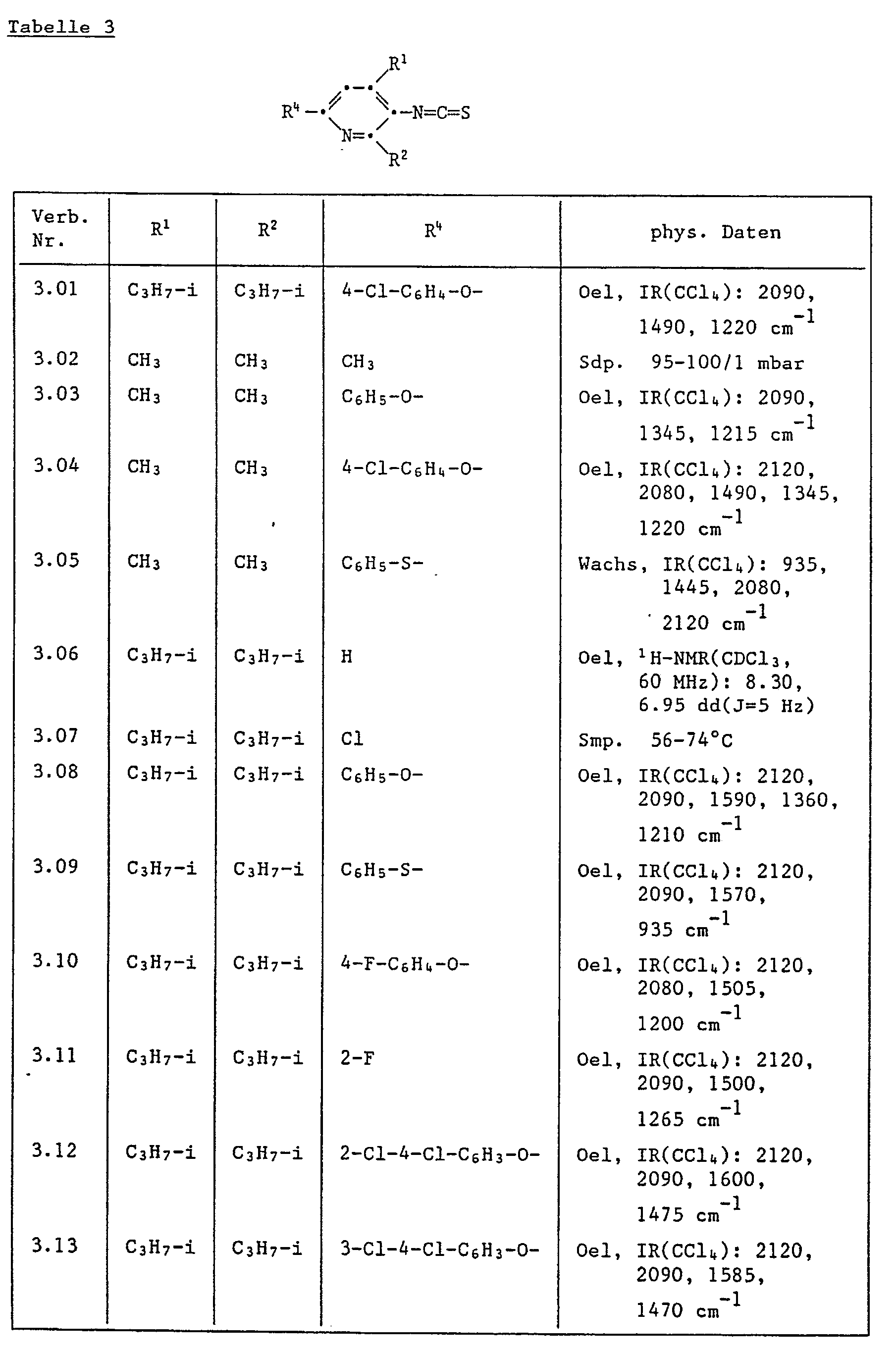

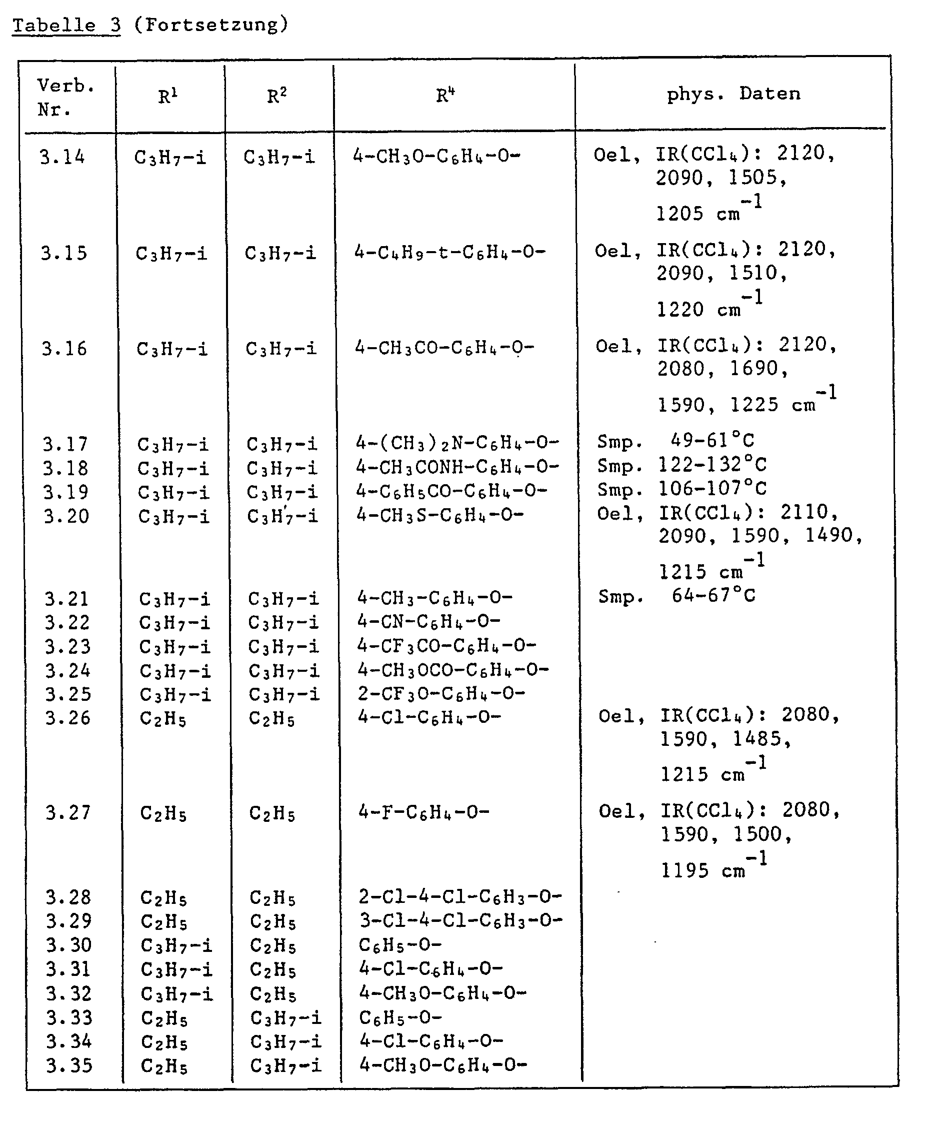

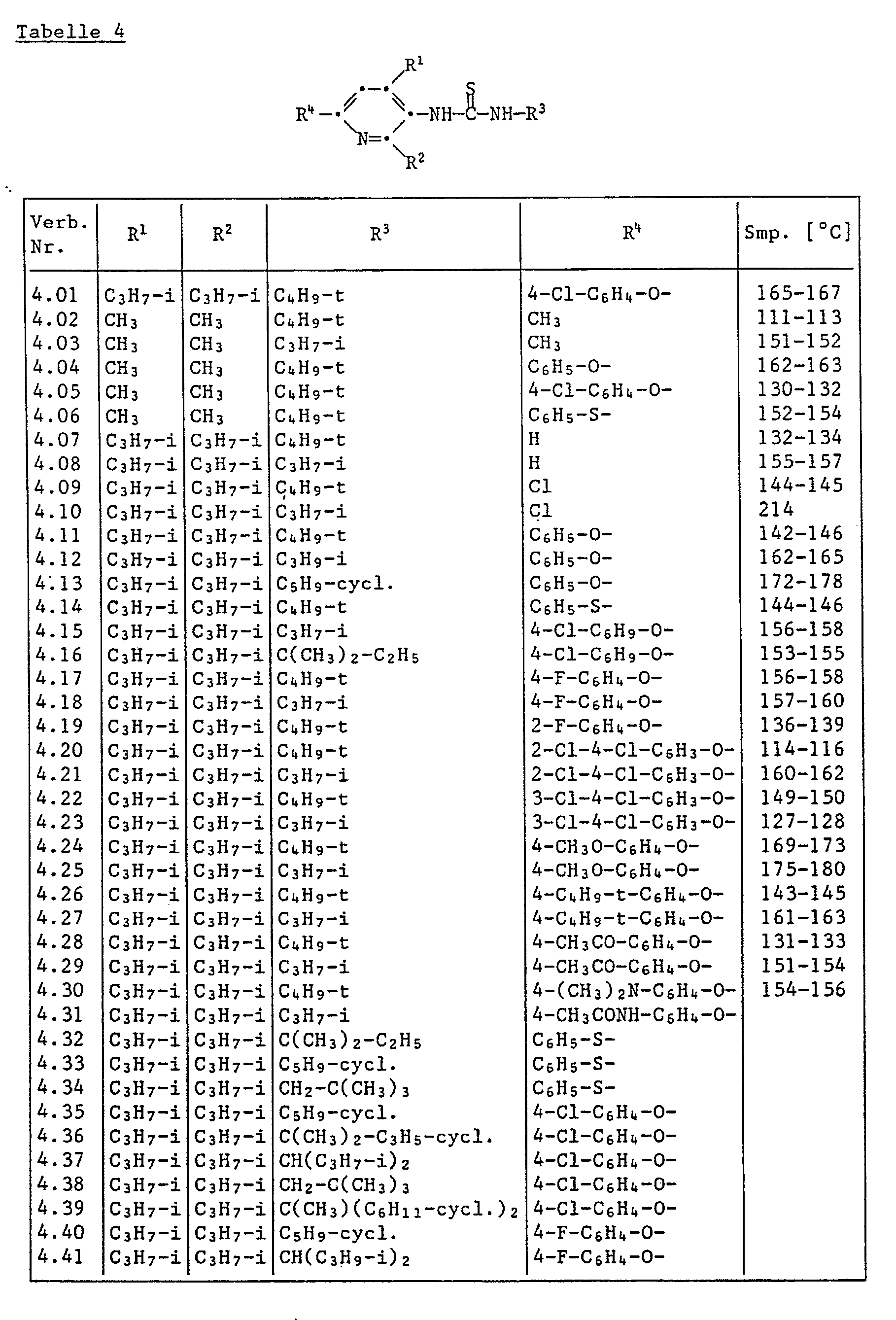

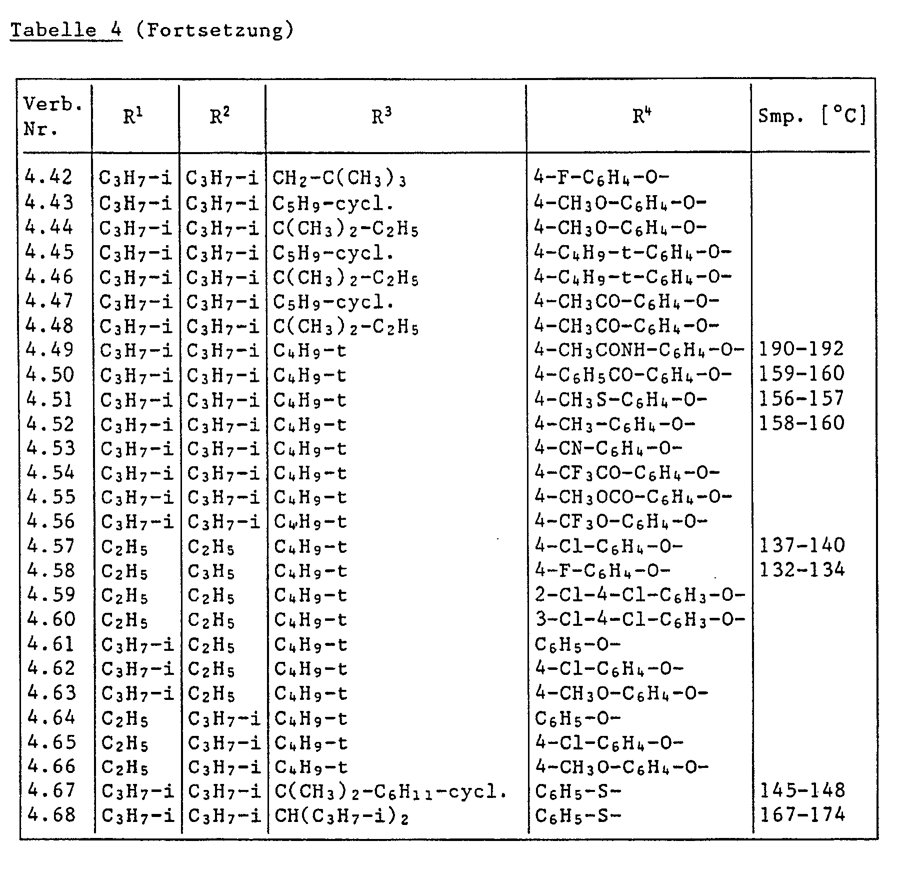

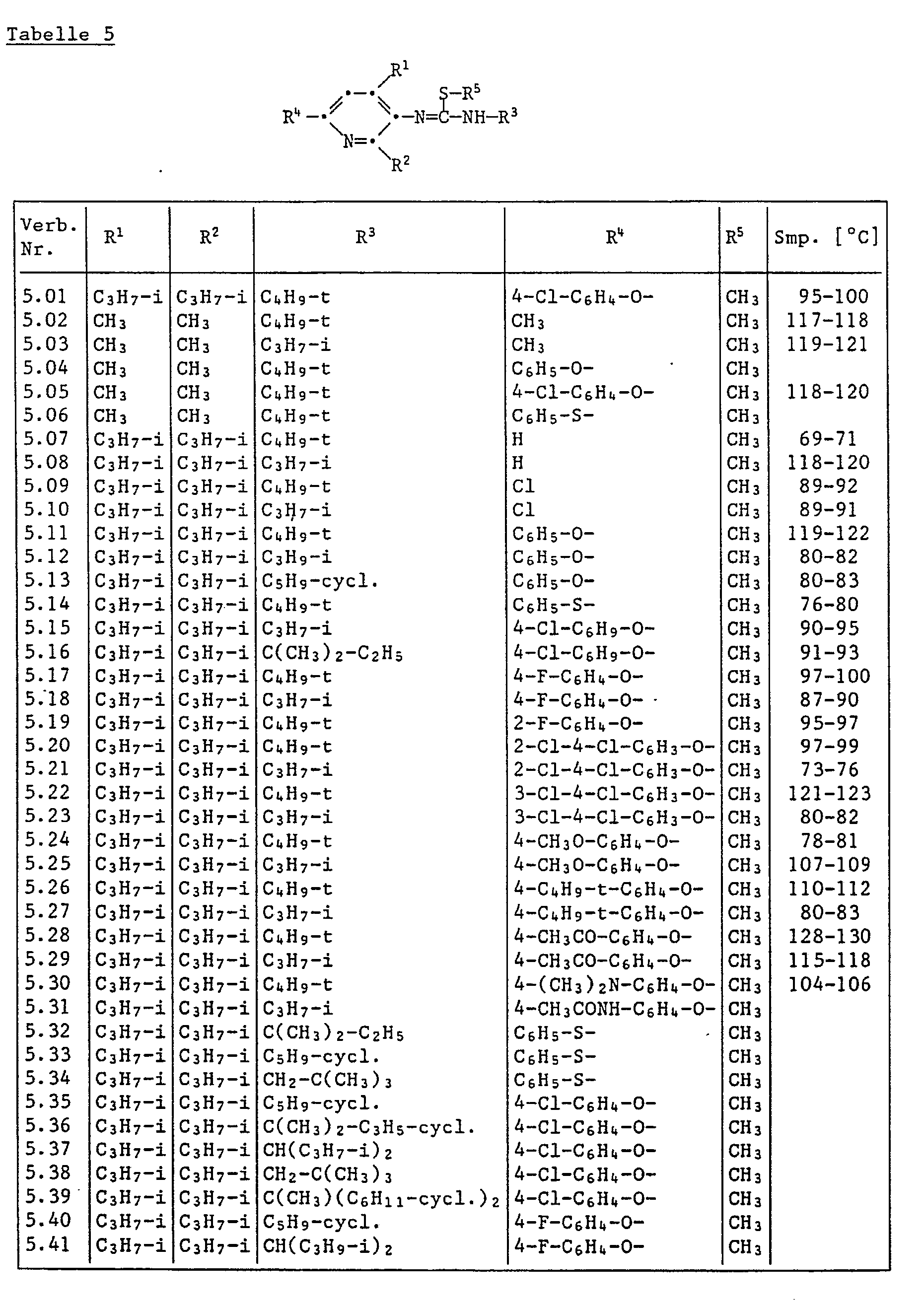

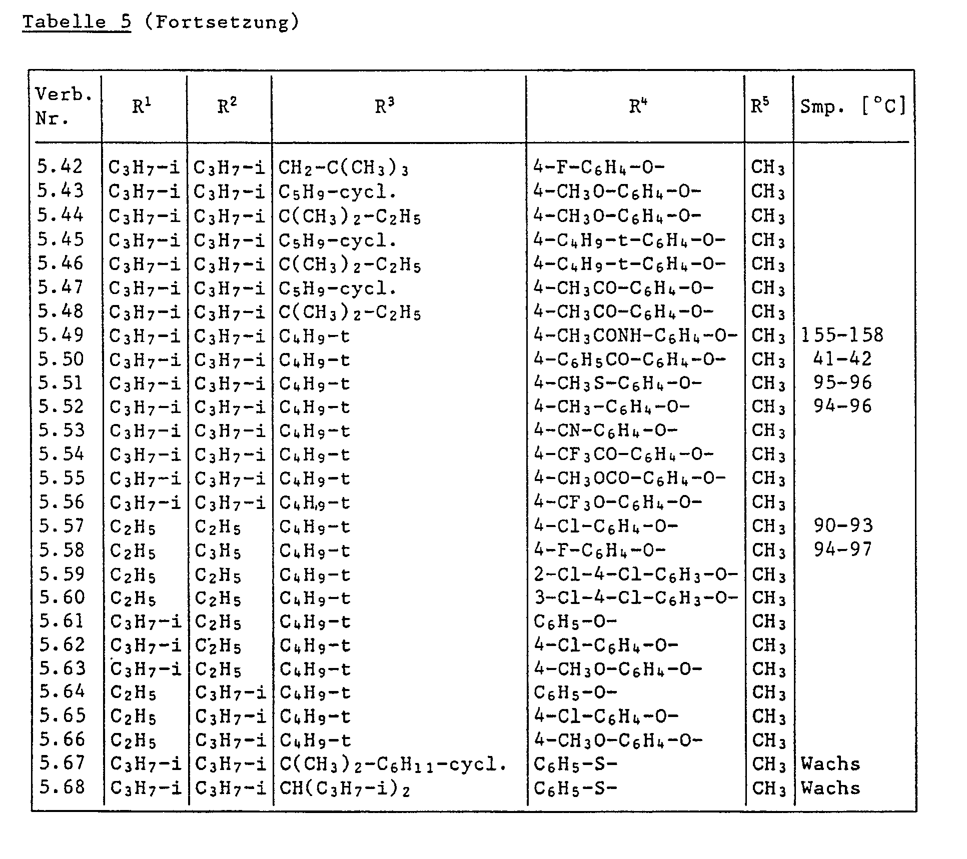

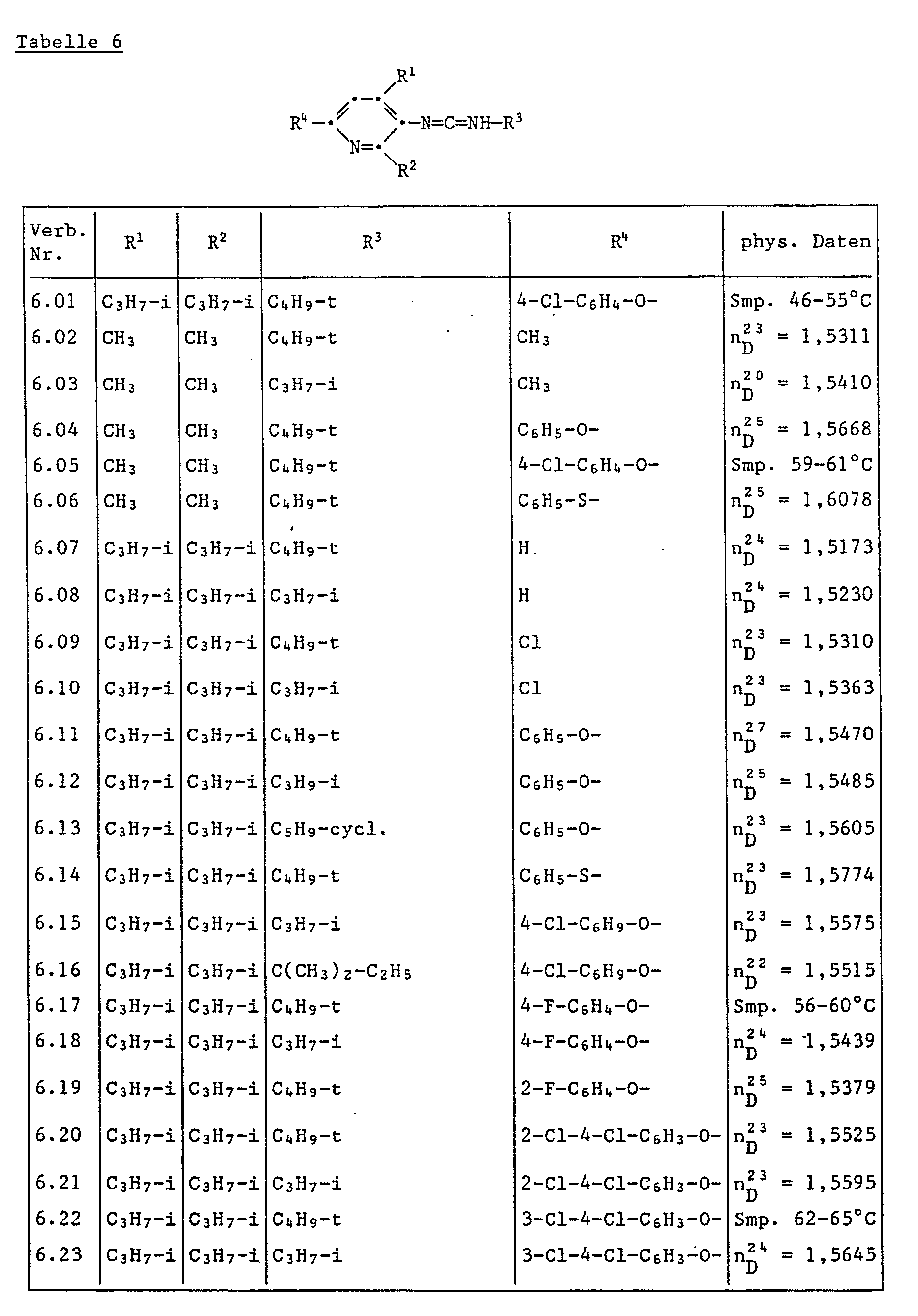

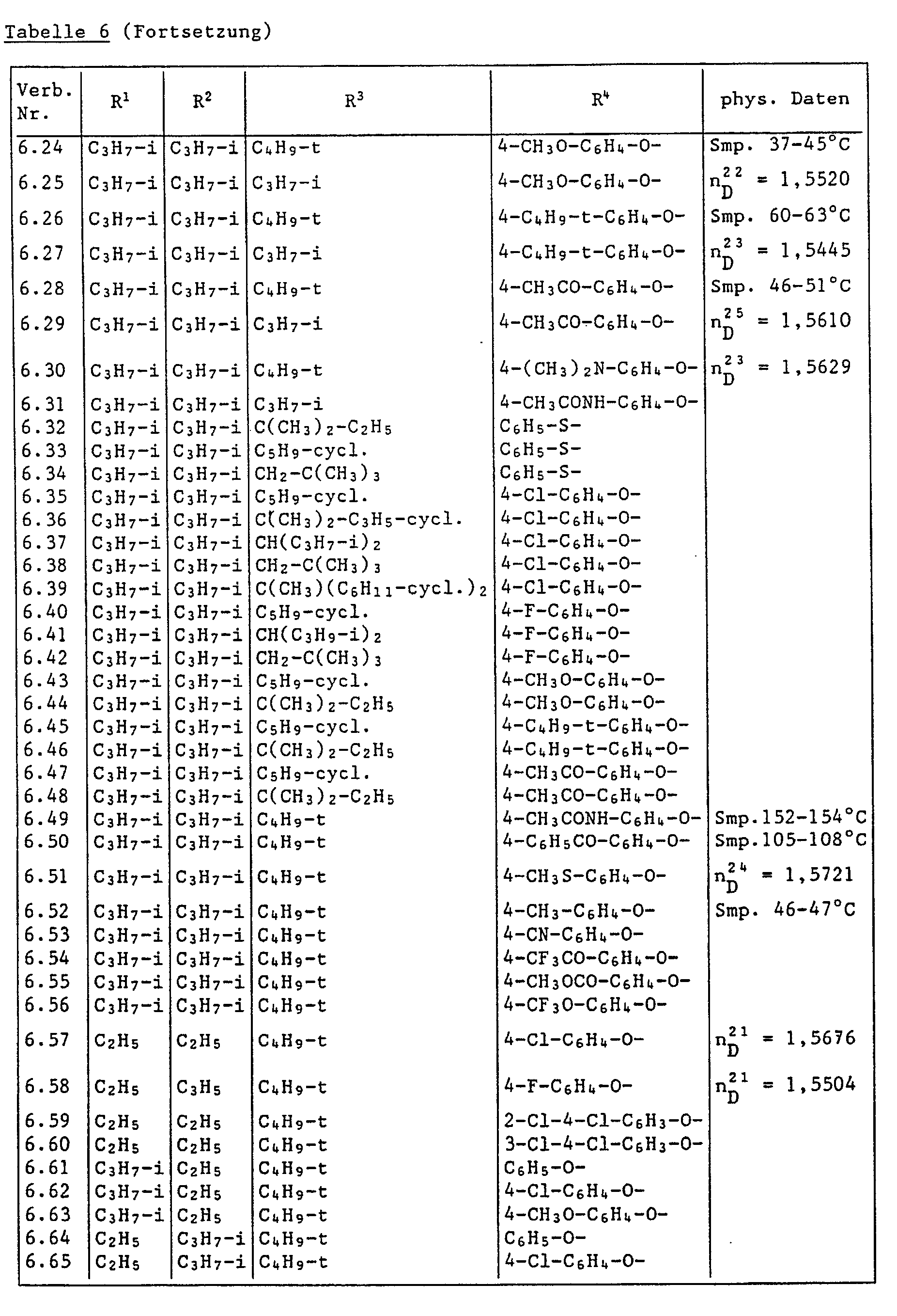

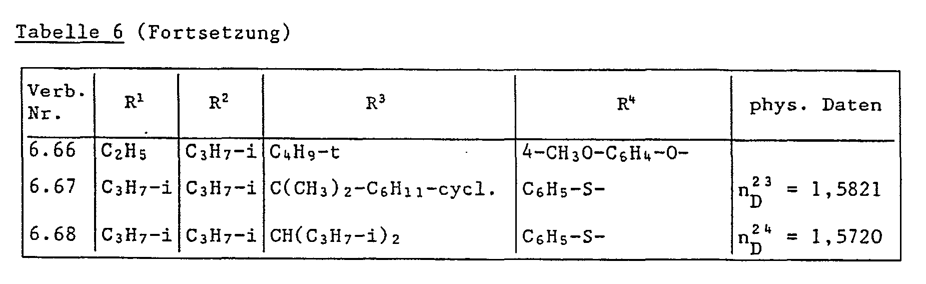

Formulierungsbeispiele (% = Gewichtsprozent)

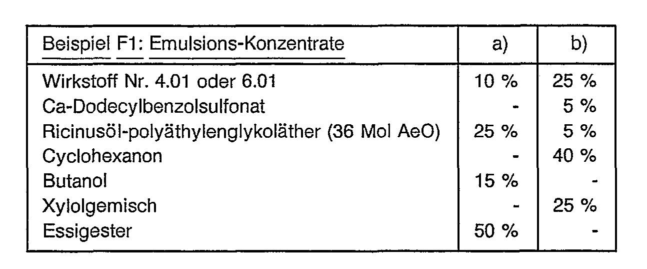

Aus solchen Konzentraten können durch Verdünnen mit Wasser Emulsionen jeder gewünschten Konzentration hergestellt werden.

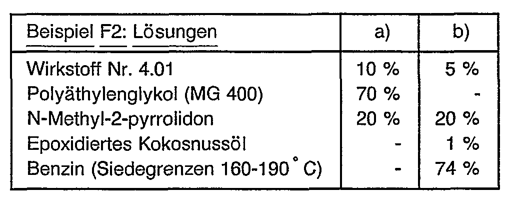

Die Lösungen sind zur Anwendung in Form kleinster Tropfen geeignet.

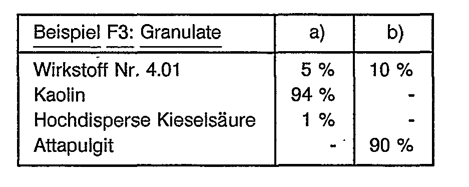

Der Wirkstoff wird in Methylenchlorid gelöst, auf den Träger aufgesprüht und das Lösungsmittel anschliessend im Vakuum abgedampft.

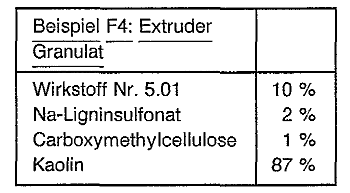

Der Wirkstoff wird mit den Zusatzstoffen vermischt, vermahlen und mit Wasser angefeuchtet. Dieses Gemisch wird extrudiert und anschliessend im Luftstrom getrocknet.

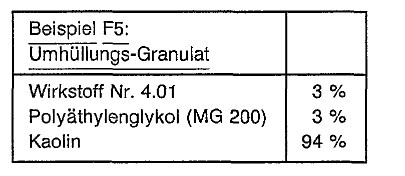

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

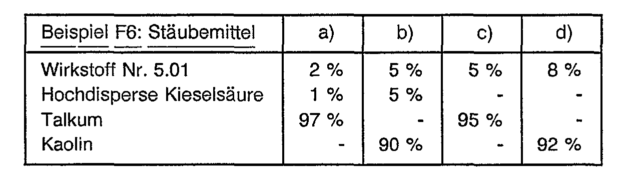

Durch inniges Vermischen der Trägerstoffe mit dem Wirkstoff und gegebenenfalls Vermahlen auf einer geeigneten Mühle erhält man gebrauchsfertige Stäubemittel.

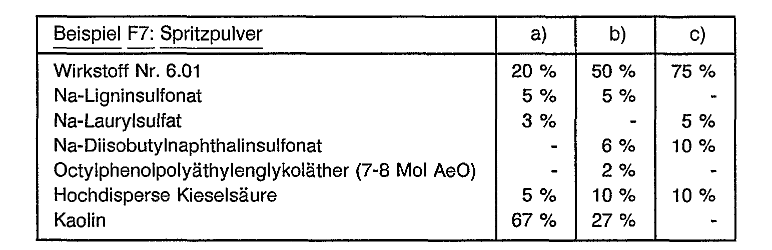

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

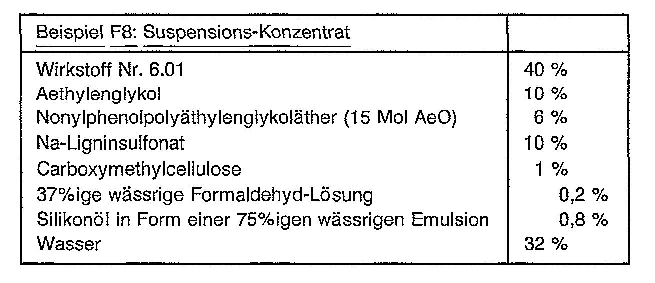

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Biologische Beispiele

### Beispiel B1: Wirkung gegen Boophilus microplus

Vollgesogene adulte Zecken Weibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Die Verbindungen gemäss Tabellen 4, 5, 6 zeigen in diesem Test gute Wirkung gegen Boophilus microplus. Insbesondere die Verbindungen 4.01, 4.11, 4.16, 4.51, 4.52, 5.01, 5.12, 5.13, 5.15, 5.16, 5.20, 5.22, 5.23, 5.50, 5.51, 5.52, 6.01, 6.07, 6.11, 6.12, 6.13, 6.15, 6.21, 6.22, 6.23, 6.24, 6.26, 6.30, 6.50, 6.51 und 6.52 zeigen eine Wirkung über 80 %.

### Beispiel B2: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.

Die Verbindungen der Tabellen 4, 5, 6 zeigen eine gute Wirkung gegen Diabrotica balteata in diesem Test. Insbesondere die Verbindungen 4.11, 4.20, 4.22, 4.24, 5.01, 5.11, 5.12, 5.15, 5.25 und 5.27 zeigen eine Wirkung über 80%.

### Beispiel B3: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm des Wirkstoffes enthält, besprüht. Die Pflanzen werden anschliessend für 6 Tage bei 25 ° C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.

Die Verbindungen der Tabellen 4, 5, 6 zeigen eine gute Wirkung gegen Tetranychus urticae in diesem Test. Insbesondere die Verbindungen 4.01, 4.04, 4.12, 4.20, 4.23, 4.24, 4.27, 4.50, 4.51, 4.52, 5.11, 5.13, 5.23, 5.24, 5.50, 5.51, 5.52, 6.06, 6.09, 6.15, 6.23, 6.29, 6.50, 6.51 und 6.52 zeigen eine Wirkung über 80 %.

### Beispiel B4: Wirkung gegen Spodoptera littoralis Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des dritten Stadiums von Spodoptera littoralis besiedelt und in einen Plastikbehälter gegeben. 3 Tage später erfolgt die Auswertung. Aus dem Vergleich der Anzahl toter Raupen und des Frassschadens auf den behandelten zu denjenigen auf den unbehandelten Pflanzen wird die prozentuale Reduktion der Population bzw. die prozentuale Reduktion des Frassschaden (% Wirkung) bestimmt.

Die Verbindungen der Tabellen 4, 5, 6 zeigen eine gute Wirkung gegen Spodoptera littoralis in diesem Test. Insbesondere die- Verbindungen 4.01, 4.11, 4.17, 4.21, 4.26, 4.50, 4.51, 5.09, 5.15, 5.16, 5.18, 5.22, 5.26, 5.51, 5.52, 6.01, 6.09, 6.12, 6.17, 6.21, 6.22, 6.24, 6.26, 6.30, 6.50, 6.51 und 6.52 zeigen eine Wirkung über 80 %.

### Beispiel B5: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten des Anthonomus grandis besiedelt und in einen Plastikbehälter gegeben. 3 Tage später erfolgt die Auswertung. Aus dem Vergleich der Anzahl toter Käfer und des Frassschadens auf den behandelten zu denjenigen auf den unbehandelten Pflanzen wird die prozentuale Reduktion der Population bzw. die prozentuale Reduktion des Frassschaden (% Wirkung) bestimmt.

Die Verbindungen der Tabellen 4, 5, 6 zeigen eine gute Wirkung gegen Anthonomus grandis in diesem Test. Insbesondere die Verbindungen 4.11, 5.01, 5.11, 5.17, 5.22 und 5.23 zeigen eine Wirkung über 80 %.

### Beispiel B6: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20 C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (%-Wirkung) bestimmt.

Die Verbindungen der Tabellen 4, 5, 6 zeigen eine gute Wirkung gegen Aphis craccivora in diesem Test. Insbesondere die Verbindungen 4.11, 5.01, 5.15, 5.16, 6.01, 6.09, 6.11, 6.12, 6.13, 6.16, 6.17, 6.20, 6.22, 6.24, 6.26, 6.28 und 6.30 zeigen eine Wirkung über 80 %.

### Beispiel B7: Wirkung gegen Myzus persicae

Erbsenkeimlinge werden mit Myzus persicae infiziert und anschliessend mit einer Spritzbrühe, die 400 ppm des Wirkstoffes enthält, besprüht und bei 20 C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (%-Wirkung) bestimmt.

Die Verbindungen der Tabellen 4, 5, 6 zeigen eine gute Wirkung gegen Myzus persicae in diesem Test. Insbesondere die Verbindungen 6.09, 6.12 und 6.13 zeigen eine Wirkung über 80 %.

### Beispiel B8: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20° C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (%-Wirkung) bestimmt.

Die Verbindungen der Tabellen 4, 5, 6 zeigen eine gute Wirkung gegen Myzus persicae in diesem Test. Insbesondere die Verbindungen 4.11, 6.01, 6.09, 6.11, 6.12, 6.13, 6.16, 6.17, 6.24, 6.26, 6.28 und 6.30 zeigen eine Wirkung über 80 %.

### Beispiel B9: Wirkung gegen Musca domestica

Ein Zuckerwürfel wird- derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25⁰ C und 50 % Luftfeuchtigkeit belassen. Nach 24 Stunden wird die insektizide Wirkung durch Ermittlung der Abtötungsrate festgestellt.

Die Verbindungen der Tabellen 4, 5, 6 zeigen in diesem Test eine gute Wirkung gegen Musca domestica. Insbesondere die Verbindungen 5.01, 6.01 und 6.07 zeigen eine Wirkung über 80 %.

### Beispiel B10: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Tabellen 4, 5 und 6 zeigen eine gute Wirkung gegen Nilaparvata lugens in diesem Test. Insbesondere die Verbindungen 4.11, 5.09, 5.11, 5.12, 6.01, 6.09, 6.11, 6.12, 6.16, 6.20, 6.21 und 6.22 zeigen eine Wirkung über 80 %.

### Beispiel B11: Systemische Wirkung gegen Nilaparvata lugens

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Die Verbindungen der Tabellen 4, 5 und 6 zeigen eine gute Wirkung gegen Nilaparvata lugens in diesem Test. Insbesondere die Verbindungen 5.09, 5.11, 5.15, 5.17, 6.09, 6.10, 6.11, 6.16, 6.17, 6.20, 6.22, 6.24 und 6.26 zeigen eine Wirkung über 80 %.

### Beispiel B12: Wirkung gegen Tetranychus urticae

Junge Bohnenpflanzen werden mit Weibchen von Tetranychus urticae besiedelt, die nach 24 Stunden wieder entfernt werden. Die mit Eiern besiedelten Pflanzen werden mit einer wässrigen Emulsions-Spritzbrühe, die 400 ppm des Wirkstoffes enthält, besprüht. Die Pflanzen werden anschliessend für 6 Tage bei 25 °C inkubiert und anschliessend ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.

Die Verbindungen der Tabellen 4, 5 und 6 zeigen eine gute Wirkung gegen Tetranychus urticae in diesem Test. Insbesondere die Verbindungen 4.11, 4.16, 4.18, 4.22, 4.26, 4.50, 4.51, 4.52, 5.15, 5.18, 5.21, 5.51, 6.13, 6.16, 6.22, 6.24, 6.30, 6.50, 6.51 und 6.52 zeigen eine Wirkung über 80 %.

### Beispiel B13: Wirkung gegen Panonychus ulmi(OP und Carb. resistent)

Apfelsämlinge werden mit adulten Weibchen von Panonychus ulmi be-siedelt. Nach sieben Tagen werden die infizierten Pflanzen mit einer wässrigen Emulsion-Spritzbrühe, enthaltend 400 ppm der zu prüfenden Verbindung, bis zur Tropfennässe besprüht und im Gewächshaus kultiviert. Nach 14 Tagen erfolgt die Auswertung. Aus dem Vergleich der Anzahl toter Spinnmilben auf den behandelten zu denjenigen auf den unbehandelten Pflanzen wird die prozentuale Reduktion an Population (% Wirkung) bestimmt.

Die Verbindungen der Tabellen 4, 5 und 6 zeigen gute Wirkung gegen Panonychus ulmi in diesem Test. Insbesondere die Verbindungen 4.16, 4.24, 4.26, 4.52, 6.28 und 6.50 zeigen eine Wirkung über 80 %.

### Beispiel B14: Wirkung gegen Dermanyssus gallinae

In einem nach oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben durch Auszählen der toten Individuen ermittelt.

Die Verbindungen der Tabellen 4, 5 und 6 zeigen eine gute Wirkung gegen Dermanyssus gallinae in diesem Test. Insbesondere die Verbindungen 4.07, 5.07, 5.16, 5.51, 6.07, 6.11 und 6.52 zeigen eine Wirkung über 80 %.

### Beispiel B15: Wirkung gegen Blattella germanica

In eine Petri-Schale wird so viel einer 0,1%igen acetonischen Lösung des Wirkstoffes gegeben, dass die Menge einer Aufwandmenge von 2 g/m² entspricht. Wenn das Lösungsmittel verdunstet ist, werden 10 Blattella germanica Nymphen (letztes Nymphenstadium) in die so vorbereitete Schale gegeben und 2 Stunden lang der Wirkung der Testsubstanz ausgesetzt. Dann werden die Nymphen mit CO₂ narkotisiert, in eine frische Petri-Schale gebracht und im Dunkeln bei 25 C und 50 bis 70 % Luftfeuchtigkeit gehalten. Nach 48 Stunden wird die insektizide Wirkung durch Ermittlung der Abtötungsrate bestimmt.

Die Verbindungen der Tabellen 4, 5 und 6 zeigen gute Wirkung gegen Blattella germanica in diesem Test. Insbesondere die Verbindungen 6.01, 6.12, 6.13 und 6.15 zeigen eine Wirkung über 80 %.

## Patentansprüche

1. 3-Amino-2,4-dialkylpyridin-Derivate der Formel I 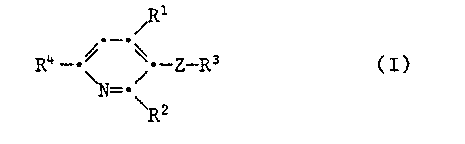 worin
R' und R² unabhängig voneinander C₁-C₆-Alkyl,
R³ C₁-C₁₂-Alkyl, Cₛ-C₆-Cycloalkyl, durch Cₛ-C₆-Cycloalkyl substituiertes C₁-C₄-Alkyl oder durch C₁-C₄-Alkyl substituiertes Cs-C₆-Cycloalkyl,
R⁴ Wasserstoff, Halogen, Cᵢ-C₆-Alkyl, Phenoxy oder einfach oder zweifach durch Halogen, C₁-C₄-Alkyl, C₁-C₄-Alkoxy, C₁-C₄-Alkylthio, C₁-C₄-Alkylamino, Di-Ci-C₄-alkylamino, C₁-C₄-Alkylcarbonylamino, C₁-C₄-Alkylcarbonyl, Benzoyl, Nitro, Cyan, C₁-C₄-Alkoxycarbonyl, C₁-C₄-Halogenalkyl oder C₁-C₄-Halogenalkoxy substituiertes Phenoxy und
Z ein Brückenglied -NH-CS-NH-, -N=C(SR⁵)-NH-oder -N=C=N- bedeuten, wobei R⁵ für C₁-C₆-Alkyl oder C₃-C₅-Alkenyl steht.

2. Verbindungen gemäss Anspruch 1, dadurch gekennzeichnet, dass R¹ und R² die gleiche Bedeutung haben.

3. Verbindungen gemäss Anspruch 1, dadurch gekennzeichnet, dass R' und R² für Isopropyl stehen.

4. Verbindungen gemäss Anspruch 1, dadurch gekennzeichnet, dass R³ für verzweigtes C₃-C₅-Alkyl steht.

5. Verbindungen gemäss Anspruch 4, dadurch gekennzeichnet, dass R³ Isopropyl, tert.Butyl oder 1,1-Dimethylpropyl bedeutet.

6. Verbindungen gemäss Anspruch 1, dadurch gekennzeichnet, dass R⁴ für durch Halogen, Ci-C₄-Alkyl oder C₁-C₄-Alkoxy substituiertes Phenoxy steht.

7. Verbindungen gemäss Anspruch 1, dadurch gekennzeichnet, dass R⁴ für Chlorphenoxy, Fluorphenoxy, Methoxyphenoxy, tert.Butylphenoxy oder Dichlorphenoxy steht.

8. Verbindungen gemäss Anspruch 1, dadurch gekennzeichnet, dass R¹ und R² die gleiche Bedeutung haben, R³ für verzweigtes C₃-C₅-Alkyl und R⁴ für durch Halogen, C₁-C₄-Alkyl, Di-C₁-C₄-alkylamino oder C₁-C₄-Alkoxy substituiertes Phenoxy stehen.

9. Verbindungen gemäss Anspruch 1, dadurch gekennzeichnet, dass R' und R² für Isopropyl, R³ für Isopropyl, tert.Butyl oder 1,1-Dimethylpropyl und R⁴ für Chlorphenoxy, Fluorphenoxy, Dichlorphenoxy, Methoxyphenoxy oder tert.Butylphenoxy stehen.

10. Verbindungen gemäss Anspruch 1, dadurch gekennzeichnet, dass R⁵ für C₁-C₄-Alkyl steht.

11. Verbindungen gemäss Anspruch 1, ausgewählt aus der Reihe
1-tert.Butyl-3-[2,4-diisopropyl-6-(4-chlorphenoxy)-pyrid-3-yl]-thioharnstoff,
1-tert.Butyl-3-[2,4-diisopropyl-6-(4-chlorphenoxy)-pyrid-3-yl]-S-methyl-isothioharnstoff,
1-tert.Butyl-3-[2,4-diisopropyl-6-(4-chlorphenoxy)-pyrid-3-yl]-carbodiimid,
1-tert.Butyl-3-[2,4-diisopropyl-6-(4-methoxyphenoxy)-pyrid-3-yl]-thioharnstoff,
1-tert.Butyl-3-[2,4-diisopropyl-6-(4-methoxyphenoxy)-pyrid-3-yl]-S-methyl-isothioharnstoff und
1-tert.Butyl-3-[2,4-diisopropyl-6-(4-methoxyphenoxy)-pyrid-3-yl]-carbodiimid.

12. Verfahren zur Herstellung der Verbindungen der Formel I, dadurch gekennzeichnet, dass man
a) ein Isothiocyanat der Formel II 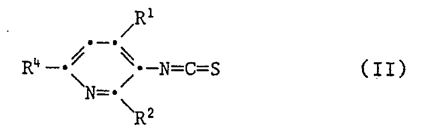 worin R', R² und R⁴ die unter Formel I gegebenen Bedeutungen haben, mit einem primären Amin der Formel III H₂N-R³ (III) worin R³ die unter Formel I gegebene Bedeutung hat zum Thioharnstoff der Formel la 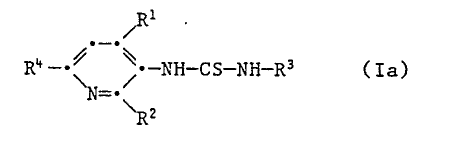 umsetzt und gewünschtenfalls entweder
b) den erhaltenen Thioharnstoff der Formel la mit einem Alkylierungsmittel der Formel IV Y-R⁵ (IV) worin R⁵ die unter Formel I gegebene Bedeutung hat und Y für eine Abgangsgruppe steht, in den Isothioharnstoff der Formel Ib 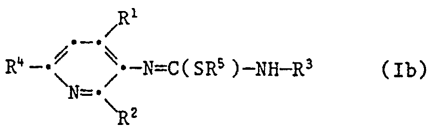 umsetzt oder
c) den erhaltenen Thioharnstoff der Formel la durch Abspalten von Schwefelwasserstoff in das Carbodiimid der Formel Ic 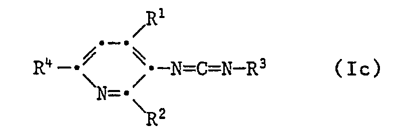 überführt.

13. Schädlingsbekämpfungsmittel, welches als aktive Komponente mindestens eine Verbindung der Formel I nach Anspruch 1 enthält.

14. Mittel gemäss Anspruch 13, dadurch gekennzeichnet, dass es zusätzlich mindestens einen Trägerstoff enthält.

15. Verwendung einer Verbindung der Formel I nach Anspruch 1 zur Bekämpfung von Schädlingen an Tieren und Pflanzen.

16. Verwendung gemäss Anspruch 15, dadurch gekennzeichnet, dass es sich bei den Schädlingen um pflanzenschädigende Insekten und Arachniden handelt.

17. Verfahren zur Bekämpfung von tier- und pflanzenschädigenden Insekten und Arachniden, dadurch gekennzeichnet, dass man die Schädlinge oder ihren Lebensraum mit einer wirksamen Menge einer Verbindung der Formel I gemäss Anspruch behandelt.

18. Pyridylisothiocyanate der Formel II 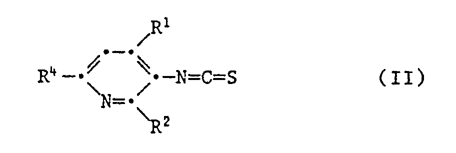 worin
R¹ und R² unabhängig voneinander C₁-C₆-Alkyl, und
R⁴ Wasserstoff, Halogen, C₁-C₆-Alkyl, Phenoxy oder einfach oder zweifach durch Halogen, C_{I}1C₄-Alkyl, C₁-C₄-Alkoxy, C₁-C₄-Alkylthio, C₁-C₄-Alkylamino, Di-C₁-C₄-alkylamino, C₁-C₄-Alkylcarbonylamino, C₁-C₄-Alkyl- car bonyl, Benzoyl, Nitro, Cyan, C₁-C₄-Alkoxycarbonyl, C₁-C₄-Halogenalkyl oder Ci-C₄-Halogenalkoxy substituiertes Phenoxy bedeuten.

19. 3-Aminopyridine der Formel V 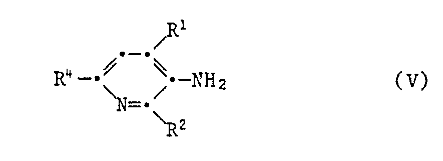 worin
R¹ und R² unabhängig voneinander C₁-C₆-Alkyl, und
R⁴ Wasserstoff, Halogen, Cᵢ-C₆-Alkyl, Phenoxy oder einfach oder zweifach durch Halogen, C₁-C₄-Alkyl, C₁-C₄-Alkoxy, C₁-C₄-Alkylthio, C₁-C₄-Alkylamino, Di-C₁-C₄-alkylamino, C₁-C₄-Alkylcarbonylamino, C₁-C₄-Alkylcarbonyl, Benzoyl, Hitro, Cyan, C₁-C₄-Alkoxycarbonyl, C₁-C₄-Halogenalkyl oder C₁-C₄-Halogenalkoxy substituiertes Phenoxy bedeuten, mit der Massgabe, dass R', R² und R⁴ nicht gleichzeitig jeweils für Methyl stehen.

20. 3-Nitropyridine der Formel VI 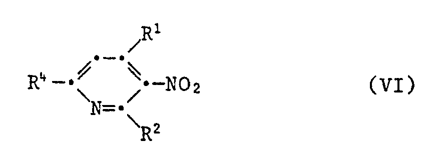 worin
R¹ und R² unabhängig voneinander C₁-C₆-Alkyl, und
R⁴ Wasserstoff, Halogen, C₁-C₆-Alkyl, Phenoxy oder einfach oder zweifach durch Halogen, C₁-C₄-Alkyl, Cᵢ-C₄-Alkoxy, C₁-C₄-Alkylthio, C₁-C₄-Alkylamino, Di-Cᵢ-C₄-alkylamino, C₁-C₄-Alkylcarbonylamino, C₁-C₄-Alkylcarbonyl, Benzoyl, Nitro, Cyan, C₁-C₄-Alkoxycarbonyl, C₁-C₄-Halogenalkyl oder C₁-C₄-Halogenalkoxy substituiertes Phenoxy bedeuten, mit der Massgabe, dass R', R² und R⁴ nicht gleichzeitig jeweils für Methyl stehen.
